# EUROPÄISCHE PATENTSCHRIFT

(11) **EP 0 964 846 B1**
(45) Veröffentlichungstag und Bekanntmachung des Hinweises auf die Patenterteilung: **16.01.2002**
(21) Anmeldenummer: 98913570.2
(22) Anmeldetag: 20.02.1998
(51) Int. Cl.: C07C 209/68, C07C 209/78, C07C 211/50

(54) **VERFAHREN ZUR HERSTELLUNG VON AROMATISCHEN POLYAMINGEMISCHEN**
PROCESS FOR PREPARING AROMATIC POLYAMINE MIXTURES
PROCEDE DE PREPARATION DE MELANGES DE POLYAMINES AROMATIQUES

(30) Priorität: 24.02.1997 DE 19707255; 09.06.1997 DE 19724213; 09.06.1997 DE 19724237
(43) Veröffentlichungstag der Anmeldung: 22.12.1999
(60) Teilanmeldung: 01109422.4
(73) Patentinhaber: BASF AKTIENGESELLSCHAFT, 67056 Ludwigshafen (DE)
(72) Erfinder: BECKER, Rainer, D-67098 Bad Dürkheim (DE); ELLER, Karsten, D-67061 Ludwigshafen (DE); LANGENSIEPEN, Hans-Werner, D-67240 Bobenheim-Roxheim (DE); HESSE, Michael, D-67549 Worms (DE)
(74) Vertreter: Isenbruck, Günter, Dr.
(86) Internationale Anmeldenummer: EP9800981
(87) Internationale Veröffentlichungsnummer: WO9837124

(56) Entgegenhaltungen:
- EP-A- 0 109 931
- EP-A- 0 264 744
- DE-A- 1 493 431
- DE-B- 1 230 033
- GB-A- 1 207 377
- GB-A- 2 066 809
- US-A- 3 362 979
- US-A- 4 039 580
- US-A- 4 071 558
- US-A- 4 286 107

## Beschreibung

Die Erfindung betrifft ein Verfahren zur Herstellung von aromatischen Polyamingemischen aus gegebenenfalls substituiertem Anilin und Formaldehyd oder Formaldehyd-Vorläufern oder einem Kondensationsprodukt dieser Verbindungen.

Diaminodiarylmethane, die gegebenenfalls substituiert sind, sind wertvolle Vorprodukte vor allem für die Herstellung von Kunststoffen. Vor allem das unsubstituierte Diaminodiphenylmethan (häufig als Methylendianilin, MDA bezeichnet) wird technisch in sehr großen Mengen hergestellt und größtenteils, nach Phosgenierung zu Methylendiphenyldiisocyanat (MDI), für die Herstellung von Polyurethanen eingesetzt. Dabei wird vorzugsweise das 4,4'- Isomere verwendet, jedoch entstehen in den bekannten Herstellungsverfahren auch die 2,4'- und 2,2'-Isomeren. Zudem werden höherkondensierte, mehrkernige Verbindungen gebildet. Die Herstellung erfolgt in der Regel aus Anilin und Formaldehyd in Gegenwart von Katalysatoren.

In den technisch ausgeübten Verfahren werden anorganische Säuren in gelöster Form als homogene Katalysatoren eingesetzt. Bevorzugt wird dabei wäßrige Salzsäure verwendet. Diese Reaktionsführung führt durch die Aufarbeitung bedingt zum Verbrauch äquimolarer Mengen an Basen, da die Säuren bei der Isolierung der gewünschten Polyamine noch neutralisiert werden müssen. Dieser Vorgang ist daher zwangsläufig mit einem Anfall entsprechend hoher Salzmengen verbunden, die entsorgt werden müssen oder aufwendig zurückgeführt werden. Ferner ist die mit der Verwendung wäßriger Säuren verbundene Korrosionsproblematik ein wesentlicher Nachteil dieses Verfahrens.

Aus diesem Grunde gab es zahlreiche Überlegungen und Versuche, um die wäßrigen Homogenkatalysatoren durch saure Heterogenkontakte zu ersetzen. Neben sauren Ionentauschern wurde die Verwendung von sauren synthetischen oder natürlichen Silicium- oder Aluminiumoxiden, wie Zeolithen oder Tonmineralien vorgeschlagen. In DE-A-1 230 033, DE-A-1 493 431, US 3,362,979, US 4,071,558, US 4,039,580, US 4,039,581 und US 4,294,987 sind entsprechende Katalysatoren beschrieben. In US 3,362,979 werden Katalysatoren wie Wolfram-, Vanadium- und Titan oxide als ineffektiv bezeichnet.

Gemäß US 4,294,987 wird in einem derartigen Verfahren die Kondensation in Gegenwart einer starken wäßrigen Säure durchgeführt, wonach durch Lösungsmittelextraktion die Säure entfernt wird. Die Umlagerung wird wiederum in Gegenwart von starker Säure, die in geringerer Menge eingesetzt wird, durchgeführt. Auch Diatomeenerde, Ton oder Zeolithe können in dieser Stufe eingesetzt werden.

Gemäß DE-A-1 230 033 wird siliciumhaltiger Ton, ein synthetischer Siliciumdioxid-Aluminiumoxid-Katalysator oder ein Magnesiumoxid-Aluminiumoxid-Katalysator eingesetzt.

Gemäß DE-A-1 493 431 wird Siliciumdioxid, Siliciumdioxid/Aluminiumoxid oder säurebehandeltes Aluminiumoxid als Katalysator eingesetzt. Bevorzugt wird Kieselgel oder bentonitartiger Ton, der Siliciumdioxid und Aluminiumoxid enthält und vorzugsweise säureaktiviert ist, eingesetzt. Neben den gewünschten Diaminodiarylmethanen fallen jedoch höhere Kondensationsprodukte in erheblichem Umfang an.

Gemäß US 4,071,558 wird ein mit Säure aktivierter Tonkatalysator, ein Siliciumdioxid-Aluminiumoxid-Crack-Katalysator oder ein Siliciumdioxid-Magnesiumoxid-Katalysator eingesetzt. Auch hier fallen bei Verwendung des Tonkatalysators größere Mengen an 2,4'-Isomeren und höhermolekularen Produkten an.

Gemäß US 4,039,580, US 4,039,581 und US 4,294,987 wird ein Gemisch aus Diaminodiarylmethanen und höherkondensierten Oligomeren in aufwendigen zweistufigen Verfahren erhalten. Neben wäßrigen Säuren werden auch Diatomeenerde, Tone oder Zeolithe eingesetzt.

Gemäß US 4,294,987 wird die Kondensation in Gegenwart einer starken wäßrigen Säure durchgeführt, wonach durch Lösungsmittelextraktion die Säure entfernt wird. Die Umlagerung wird wiederum in Gegenwart von starker Säure, die in geringerer Menge eingesetzt wird, durchgeführt. Auch Diatomeenerde, Tone oder Zeolithe können in dieser Stufe eingesetzt werden.

Gemäß US 4,039,580 wird eine Kondensation von Anilin und Formaldehyd in Abwesenheit eines Katalysators ausgeführt und das Kondensationsprodukt in Gegenwart von Diatomeenerde, Tonen oder Zeolithen umgesetzt. Der Diamingehalt im Produkt beträgt 44 bis 50 %. In der US 4,039,581 sind ähnliche Umsetzungen beschrieben. Technisch haben sich diese Katalysatoren jedoch aufgrund der hohen Preise, der nicht ausreichenden Aktivitäten oder mangelnden Katalysatorstandzeiten nicht durchsetzen können. Hohe Anteile an 4,4'-Isomeren und sehr niedrige Gehalte an höherkondensierten Produkten sind in diesen Verfahren jedoch nicht zugänglich. Es fallen immer größere Anteile an 2,4'- bzw. 2,2'- Isomeren an.

Es besteht somit weiterhin die Nachfrage nach heterogenen Katalysatoren, die kostengünstig sind, eine hohe Aktivität und lange Standzeit aufweisen, umweltverträglich sind und zu hohen Ausbeuten an 4,4'-Isomeren bei geringen Mengen an höherkondensierten Produkten führen.

Aufgabe der vorliegenden Erfindung ist die Bereitstellung von Katalysatoren, die die vorgenannten Eigenschaften aufweisen, sowie eines Verfahrens zur Herstellung von aromatischen Polyamingemischen.

Erfindungsgemäß wird die Aufgabe gelöst durch ein Verfahren zur Herstellung von aromatischen Polyamingemischen, die Verbindungen der allgemeinen Formel (I) enthalten,

H₂N-A-CH₂-B-NH₂ (I)

in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung einer Verbindung der allgemeinen Formel (IV)

H-A-NH-CH₂-HN-B-H (IV)

und/oder einer Verbindung der allgemeinen Formel (V)

H-A-NH-CH₂-B-NH₂ (V)

wobei A und B wie vorstehend substituiert sind,
bei einer Temperatur im Bereich von 20°C bis 200°C in Gegenwart eines heterogenen anorganischen Katalysators, der ausgewählt ist aus einem oder mehreren Oxiden von Elementen der 3. bis 10., vorzugsweise 4. bis 6. Gruppe des Periodensystems der Elemente, ausgenommen Vanadium, der säureaktiviert sein kann.

Dabei kann die Verbindung der allgemeinen Formeln (IV) und/oder (V) durch Umsetzung von gegebenenfalls am aromatischen Kern in o- oder m-Position mit 1 bis 4 Substituenten, ausgewählt aus C₁₋₂₀-Alkylresten oder Halogenatomen, substituiertem Anilin mit Formaldehyd oder Formaldehyd-Vorläufern erhalten werden.

Zudem wird die Aufgabe gelöst durch ein Verfahren zur Herstellung von aromatischen Polyamingemischen, die Verbindungen der allgemeinen Formel (I) enthalten,

H₂N-A-CH₂-B-NH₂ (I)

in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung von gegebenenfalls am aromatischen Kern in o- oder m-Position mit 1 bis 4 Substituenten, ausgewählt aus C₁₋₂₀-Alkylresten und Halogenatomen, substituierten Anilinen mit Formaldehyd oder Formaldehyd-Vorläufern bei einer Temperatur im Bereich von 20°C bis 200°C in Gegenwart eines heterogenen anorganischen Katalysators, der ausgewählt ist aus einem oder mehreren Oxiden von Elementen der 3. bis 10., vorzugsweise 4. bis 6. Gruppe des Periodensystems der Elemente, ausgenommen Vanadium, der säureaktiviert sein kann.

### Katalysator

Es wurde erfindungsgemäß gefunden, daß sich Oxide von Elementen der 3. bis 10., vorzugsweise 4. bis 6. Gruppe des Periodensystems der Elemente oder deren Gemische, ausgenommen Vanadium, sehr vorteilhaft als Katalysatoren in den vorstehenden Umsetzungen einsetzen lassen. Die Gruppeneinteilung des Periodensystems folgt der neuen Notation, vergleiche Cotton and Wilkinson, Advanced Inorganic Chemistry, 5. Auflage, John Wiley & Sons.

Vorzugsweise werden Oxide der 4. und/oder 6. Gruppe des Periodensystems der Elemente eingesetzt. Besonders bevorzugt werden als Katalysator Titandioxid, Wolframoxid, Molybdänoxid, Zirconiumdioxid oder Gemische dieser Oxide eingesetzt. Unter "Gemische" werden Gemische zweier oder mehrerer der vorstehend genannten Oxide verstanden. Es kann sich dabei um Gemische der einzelnen pulverförmigen Oxide handeln, oder um gemeinsame Fällungsprodukte aus Lösungen, die lösliche Verbindungen der Metalle enthalten. Verfahren zur Herstellung der erfindungsgemäß verwendeten Katalysatoren sind bekannt.

Die vorstehenden Katalysatoren können zudem säureaktiviert sein. Eine Säureaktivierung kann dabei beispielsweise durch Schwefelsäure, Phosphorsäure oder Salzsäure, vorzugsweise durch Schwefelsäure erfolgen. Die Katalysatoren können so sulfat-, phosphat- oder chloridhaltig sein.

Die vorstehenden Katalysatoren können bevorzugt sulfathaltig sein. Hierzu wird beispielsweise der Oxidkatalysator in Schwefelsäure getränkt und anschließend getrocknet. Hierdurch werden die Katalysatoren sulfathaltig. Der Gehalt an Säure, vorzugsweise Sulfat beziehungsweise Schwefelsäure kann in weiten Bereichen variieren und einfach den gewünschten Bedingungen angepaßt werden. Der Katalysator kann auch durch Tränken in säurehaltigen, vorzugsweise sulfathaltigen Lösungen, wie Alkali- oder Erdalkalimetallsalzlösungen und anschließendem Trocknen hergestellt werden.

Die Katalysatoren können pulverförmig oder in Form von Formkörpern, wie Strängen, Granulaten, Tabletten, Pellets, Kugeln eingesetzt werden. Entsprechend kann die Umsetzung in Suspensionsfahrweise oder mit Festbettkatalysatoren durchgeführt werden. Die Umsetzung kann dabei in allen Fällen diskontinuierlich oder kontinuierlich ausgeführt werden.

Gemäß einer Ausführungsform des erfindungsgemäßen Verfahrens wird gegebenenfalls am aromatischen Kern in o- oder m-Position mit 1 bis 4 Substituenten, ausgewählt aus C₁₋₂₀-, vorzugsweise C₁₋₁₀-, besonders bevorzugt C₁₋₆-, insbesondere C₁₋₃-Alkylresten und Halogenatomen, vorzugsweise Fluor-, Chlor- oder Bromatomen, insbesondere Chloratomen substituiertes Anilin mit Formaldehyd oder Formaldehyd-Vorläufern umgesetzt.

Vorzugsweise liegen im gegebenenfalls substituierten Anilin 0 bis 2, besonders bevorzugt keiner oder ein Substituent vor. Liegt ein Substituent vor, so ist es vorzugsweise ein Alkylrest, insbesondere ein Methyl-, Ethyl- oder Propylrest. Dieser Substituent liegt vorzugsweise in der o-Position vor. Bevorzugt eingesetzte Verbindungen sind Anilin oder o-Toluidin. Das gegebenenfalls substituierte Anilin wird mit Formaldehyd oder Formaldehyd-Vorläufern umgesetzt. Als Formaldehyd-Vorläufer dienen solche Verbindungen, die unter den Reaktionsbedingungen Formaldehyd freisetzen. Beispiele hierfür sind Paraformaldehyd und Trioxan.

Die Umsetzung kann dabei direkt in Gegenwart des erfindungsgemäßen Katalysators durchgeführt werden, d.h. der Katalysator wird bereits direkt zu Beginn der Umsetzung zugefügt. Dabei können beispielsweise das gegebenenfalls substituierte Anilin gemeinsam mit dem Katalysator vorgelegt und der Formaldehyd gasförmig oder als wäßrige Lösung oder als Formaldehyd-Vorläufer zudosiert werden. Die Umsetzung kann auch durchgeführt werden, indem der Formaldehyd oder der Formaldehyd-Vorläufer mit dem gegebenenfalls substituierten Anilin gemeinsam vorgelegt und der Katalysator anschließend eingetragen wird. Gemäß dieser weiteren Ausführungsform der Erfindung wird zunächst der Formaldehyd mit dem gegebenenfalls substituierten Anilin in Abwesenheit des Katalysators umgesetzt, wobei sich eine Kondensationsverbindung der allgemeinen Formel (IV) bzw. (V) bildet. Dieses Vorkondensat kann anschließend in Gegenwart des erfindungsgemäßen Katalysators umgesetzt werden, wobei die Umlagerung zu einer Verbindung der allgemeinen Formel (I) eintritt.

Das bei der Bildung des Vorkondensates entstehende Reaktionswasser kann kontinuierlich entfernt werden. Es kann auch am Ende der Umsetzung zur Bildung des Vorkondensats durch Destillation entfernt werden oder im Reaktionsgemisch verbleiben.

Wird ein gegebenenfalls substituiertes Anilin eingesetzt, bei dem nicht beide o-Positionen substituiert sind, so können bei der Isomerisierung neben den 4,4'-Isomeren der Formel (I) auch die 2,4'-Isomeren der Formel (II) und die 2,2'-Isomeren der Formel (III) gebildet werden. Die Erfindung betrifft so auch ein Verfahren, bei dem die aromatischen Kerne in mindestens einer o-Stellung zur Aminogruppe nicht substituiert sind und die aromatischen Polyamingemische ferner Verbindungen der allgemeinen Formeln (II) und /oder (III) enthalten

H₂N-D-CH₂-B-NH₂ (II)

in der D ein 1,2-Phenylenrest und B ein 1,4-Phenylenrest sind, die die vorstehenden Substituenten aufweisen können,

H₂N-D-CH₂-E-NH₂ (III)

in der D und E 1,2-Phenylenreste sind, die die vorstehenden Substituenten aufweisen können.

Durch Verwendung der erfindungsgemäßen Katalysatoren kann der Anteil der Verbindungen der Formel (I) gegenüber dem Anteil der Verbindungen der Formeln (II) und (III) gezielt variiert werden. Verbindungen der Formel (III) werden nur in sehr untergeordnetem Maße gebildet. Das Molverhältnis von Verbindungen der Formel (I) zu Verbindungen der Formel (II) beträgt vorzugsweise mehr als 4, sofern Verbindungen der Formel (II) gebildet werden können.

Die erfindungsgemäße Umsetzung, insbesondere die Umlagerungsreaktion, verläuft vorzugsweise im Temperaturbereich von 20°C bis 200°C, besonders bevorzugt von 100°C bis 150°C. Die Umsetzung kann dabei in Abwesenheit oder in Gegenwart eines Lösungsmittels durchgeführt werden. Als Lösungsmittel kommmen protische Lösungsmittel wie Alkohole, auch Diole wie Glykol, oder aprotische Lösungsmittel wie N-Methylpyrrolidon zum Einsatz. Vorzugsweise wird die Umsetzung in Abwesenheit eines Lösungsmittels durchgeführt.

Die Umsetzung wird üblicherweise drucklos durchgeführt, kann aber auch bei Unterdruck oder Überdruck durchgeführt werden. Die Umsetzungszeit beträgt - je nach Temperatur - vorzugsweise 10 Minuten bis 10 Stunden, besonders bevorzugt 0,5 bis 5 Stunden oder 1 bis 5 Stunden bei diskontinuierlicher Fahrweise. Die eingesetzte Katalysatormenge beträgt dabei 1 bis 40, vorzugsweise 5 bis 20 Gew.-%, bezogen auf das Gewicht des Vorkondensates. Bei kontinuierlicher Fahrweise wird vorzugsweise eine Katalysatorbelastung von 0,1 bis 1 l Ausgangsgemisch/l Katalysator x h gefahren. Das Molverhältnis des gegebenenfalls substituierten Anilins zum Formaldehyd beträgt vorzugsweise 2 bis 50, besonders bevorzugt 2,5 bis 10.

Zur Aufarbeitung wird das Reaktionsgemisch - bei Suspensionsfahrweise nach Abfiltrieren des Katalysatorpulvers - destillativ von gegebenenfalls vorliegendem Lösungsmittel, beziehungsweise nicht umgesetztem, gegebenenfalls substituiertem Anilin, befreit und anschließend vom gegebenenfalls anfallenden hochsiedenden Rückstand abdestilliert. Das hierbei als Destillat erhaltene Polyamingemisch kann direkt in Folgereaktionen, wie einer Phosgenierung oder auch Kernhydrierung, eingesetzt werden.

Nachstehend wird die Erfindung anhand von Beispielen näher erläutert. Dabei bedeuten die angegebenen Teile Gewichtsteile.

### Katalysator

### Beispiel 1 (diskontinuierliche Fahrweise)

3210 Teile o-Toluidin werden mit 405 Teilen 37%iger wäßriger Formaldehydlösung vermischt, das Wasser wird durch Azeotropdestillation entfernt und das verbleibende Gemisch ("Vorkondensat") mit 54 Teilen handelsüblichem Titandioxidpulver (beispielsweise VKR 611 von Sachtleben, Deutschland) versetzt. Nach Erhitzen auf 130°C ist die Umlagerung nach 30 Minuten beendet. Das rohe Reaktionsgemisch enthält - nach gaschromatographischer Analyse - neben 68% unumgesetztem Toluidin 28,3% des gewünschten Diaryldiaminomethans als Isomerengemisch mit einem Verhältnis von 4,4'-Isomer zu 2,4'-Isomer von 10,5.

### Beispiele 2 - 13

Bei diesen Beispielen wird analog Beispiel 1 mit unterschiedlichen Katalysatoren bzw. bei unterschiedlichen Bedingungen gearbeitet:

| Beispiel Nr. | Katalysator | Temp. | Umsatz nach 120 Min. | Isomerenverhältnis* |
|---|---|---|---|---|
| 2 | ZrO₂/WO₃(20%) | 130°C | 100% | 13,9 |
| 3 | TiO₂/WO₃(15%)¹ | 130°C | 100% | 15,8 |
| 4 | TiO₂/WO₃(15%)² | 130°C | 100% | 9,7 |
| 5 | ZrO₂ | 130°C | <10% | - |
| 6 | WO₃ | 130°C | ca.70% | - |
| 7 | TiO₂/SO₄(2,4%S)³ | 100°C | ca.70% | - |
| 8 | TiO₂/SO₄(2,4%S) | 130°C | 100% | 10,5 |
| 9 | TiO₂/SO₄(2,4%S) | 170°C | 100% | 4,8 |
| 10 | TiO₂/WO₃(5%) | 130°C | ca.20% | - |
| 11 | TiO₂/MoO₃(15%) | 130°C | 100% | 9,4 |
| 12 | ZrO₂/MoO₃(20%) | 130°C | 100% | 17,2 |
| 13 | ZrO₂/SO₄(3%S) | 130°C | ca.80% | - |

| | | | | |
|---|---|---|---|---|
| 1) getempert bei 650°C | | | | |
| 2) getempert bei 250°C | | | | |
| 3) % bezogen auf Schwefel im SO₄ | | | | |
| * des 4,4' zum 2,4'-Isomeren; das 2,2'-Isomer ist nicht nachweisbar | | | | |

### Beispiel 14 (kontinuierliche Fahrweise)

In den Reaktor einer kontinuierlich betriebenen Anlage werden 100 ml TiO₂ als Strang mit einem Durchmesser von 2,5 mm gefüllt. Anlagerungsprodukt (Vorkondensat), welches seinerseits hergestellt wurde aus 1600 g o-Toluidin, 203 g wäßriger Formaldehydlösung (75 g Formaldehyd berechnet als 100 %) und 1600 g Ethylenglykol als Lösungsmittel, wird mit einer Verweilzeit von ca. 20 Stunden bei 130°C kontinuierlich über das Katalysatorbett gepumpt.

*Der Rohaustrag zeigt gaschromatographisch folgende Zusammensetzung:*
- 67,4 %: o-Toluidin
- 2,7 %: 2,4'-Toluidinbase
- 29,9: 4,4'-Toluidinbase
(jeweils glykolfrei gerechnet)

## Patentansprüche

1. Verfahren zur Herstellung von aromatischen Polyamingemischen, die Verbindungen der allgemeinen Formel (I) enthalten,
H₂N-A-CH₂-B-NH₂ (I)
in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung einer Verbindung der allgemeinen Formel (IV),
H-A-NH-CH₂-HN-B-H (IV)
und/oder einer Verbindung der allgemeinen Formel (V)
H-A-NH-CH₂-B-NH₂ (V)
wobei A und B wie vorstehend definiert substituiert sind,
bei einer Temperatur im Bereich von 20°C bis 200°C in Gegenwart eines heterogenen anorganischen Katalysators, der ausgewählt ist aus
einem oder mehreren Oxiden von Elementen der 3. bis 10. Gruppe des Periodensystems der Elemente ausgenommen Vanadium, der säureaktiviert sein kann.

2. Verfahren nach Anspruch 1, **dadurch gekennzeichnet, daß** die Verbindung der allgemeinen Formeln (IV) oder (V) durch Umsetzung von gegebenenfalls am aromatischen Kern in o- oder m- Position mit 1 bis 4 Substituenten, ausgewählt aus C₁₋₂₀-Alkylresten oder Halogenatomen, substituiertem Anilin mit Formaldehyd oder Formaldehyd-Vorläufern erhalten wird.

3. Verfahren zur Herstellung von aromatischen Polyamingemischen, die Verbindungen der allgemeinen Formel (I) enthalten
H₂N-A-CH₂-B-NH₂ (I)
in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung von gegebenenfalls am aromatischen Kern in o- oder m- Position mit 1 bis 4 Substituenten, ausgewählt aus C₁₋₂₀-Alkylresten und Halogenatomen, substituierten Anilinen mit Formaldehyd oder Formaldehyd-Vorläufern bei einer Temperatur im Bereich von 20°C bis 200°C in Gegenwart eines heterogenen Katalysators, der ausgewählt ist aus
a) einem oder mehreren Oxiden von Elementen der 3. bis 10. Gruppe des Periodensystems der Elemente, ausgenommen Vanadium, der säureaktiviert sein kann.

4. Verfahren nach Anspruch 2 oder 3, **dadurch gekennzeichnet, daß** das bei der Umsetzung entstehende Wasser kontinuierlich entfernt wird.

5. Verfahren nach einem der Ansprüche 1 bis 4, **dadurch gekennzeichnet, daß** die aromatischen Kerne in mindestens einer o-Stellung zur Aminogruppe nicht substituiert sind und die aromatischen Polyamingemische ferner Verbindungen der allgemeinen Formeln (II) und/oder (III) enthalten
H₂N-D-CH₂-B-NH₂ (II)
in der D ein 1,2-Phenylenrest und B ein 1,4-Phenylenrest sind, die durch Substituenten, wie sie in Anspruch 3 definiert sind, substituiert sein können,
H₂N-D-CH₂-E-NH₂ (III)
in der D und E 1,2-Phenylenreste sind, die durch Substituenten, wie sie in Anspruch 3 definiert sind, substituiert sein können.

6. Verfahren nach Anspruch 2, 3 oder 4, **dadurch gekennzeichnet, daß** Anilin oder o-Toluidin eingesetzt werden.

7. Verfahren nach einem der Ansprüche 1 bis 6, **dadurch gekennzeichnet, daß** als Katalysator
ein oder mehrere Oxide von Elementen der 4. bis 6. Gruppe des Periodensystems der Elemente eingesetzt werden, wobei der Katalysator säureaktiviert sein kann.

8. Verfahren nach einem der Ansprüche 1 bis 7, **dadurch gekennzeichnet, daß**
als Katalysator Titandioxid, Wolframoxid, Molybdänoxid, Zirconiumdioxid oder Gemische dieser Oxide eingesetzt werden, wobei der Katalysator säureaktiviert sein kann.

9. Verfahren nach einem der Ansprüche 1 bis 8, **dadurch gekennzeichnet, daß** die Verbindung der Formel (I) als Hauptprodukt gebildet wird.

10. Verfahren nach einem der Ansprüche 1 bis 9, **dadurch gekennzeichnet, daß** die Umsetzung in Abwesenheit eines Lösungsmittels durchgeführt wird.

11. Verwendung von heterogenen anorganischen Katalysatoren, die ausgewählt sind aus
von einem oder mehreren Oxiden von Elementen der 3. bis 10. Gruppe des Periodensystems der Elemente, ausgenommen Vanadium, der säureaktiviert sein kann;
bei der Herstellung von aromatischen Polyamingemischen, wie sie in Anspruch 1 oder 5 definiert sind.

## Claims

1. A process for preparing aromatic polyamine mixtures which contain compounds of the formula (I)
H₂N-A-CH₂-B-NH₂ (I)
where A and B are 1,4-phenylene radicals each of which independently of one another can have from 1 to 4 substituents selected from C₁₋₂₀ alkyls and halogens,
by reacting a compound of the formula (IV)
H-A-NH-CH₂-HN-B-H (IV)
and/or a compound of the formula (V)
H-A-NH-CH₂-B-NH₂ (V)
where A and B are substituted as defined above,
at from 20°C to 200°C in the presence of a heterogeneous, inorganic catalyst selected from
one or more oxides of elements of group 3 to group 10 of the Periodic Table of the Elements, with the exception of vanadium which can be acid-activated.

2. A process as claimed in claim 1, wherein the compound of the formulae (IV) or (V) is obtained by reacting aniline, which is unsubstituted or substituted at the aromatic ring in the o- or m-position with from 1 to 4 substituents selected from C₁₋₂₀ alkyls or halogens, with formaldehyde or formaldehyde precursors.

3. A process for preparing aromatic polyamine mixtures which contain compounds of the formula (I)
H₂N-A-CH₂-B-NH₂ (I)
where A and B are 1,4-phenylene radicals each of which independently of one another can have from 1 to 4 substituents selected from C₁₋₂₀ alkyls and halogens,
by reacting anilines, which are unsubstituted or substituted at the aromatic ring in the o- or m-position with from 1 to 4 substituents selected from C₁₋₂₀ alkyls and halogens, with formaldehyde or formaldehyde precursors at from 20°C to 200°C in the presence of a heterogeneous catalyst
selected from one or more oxides of elements of group 3 to group 10 of the Periodic Table of the Elements, a) with the exception of vanadium, which can be acid-activated.

4. A process as claimed in claim 2 or 3, wherein the water produced in the reaction is continuously removed.

5. A process as claimed in one of claims 1 to 4, wherein the aromatic rings are unsubstituted at at least one o-position to the amino group and the aromatic polyamine mixtures in addition contain compounds of the formulae (II) and/or (III)
H₂N-D-CH₂-B-NH₂ (II)
where D is a 1,2-phenylene radical and B is a 1,4-phenylene radical, which may be substituted with substituents as defined in claim 3.
H₂N-D-CH₂-E-NH₂ (III)
where D and E are 1,2-phenylene radicals, which may be substituted with substituents as defined in claim 3.

6. A process as claimed in claim 2, 3 or 4, wherein aniline or o-toluidine is used.

7. A process as claimed in one of claims 1 to 6, wherein, as catalyst,
use is made of one or more oxides of elements of group 4 to group 6 of the Periodic Table of the Elements, where the catalyst can be acid-activated.

8. A process as claimed in one of claims 1 to 7, wherein,
as catalyst, use is made of titanium dioxide, tungsten oxide, molybdenum oxide, zirconium dioxide or mixtures of these, where the catalyst can be acid-activated.

9. A process as claimed in one of claims 1 to 8, wherein the compound of the formula (I) is formed as main product.

10. A process as claimed in one of claims 1 to 9, wherein the reaction is carried out in the absence of a solvent.

11. The use of hetergeneous, inorganic catalysts
selected from one or more oxides of elements of group 3 to group 10 of the Periodic Table of the Elements, with the exception of vanadium, which can be acid-activated;
in the preparation of aromatic polyamine mixtures as defined in claim 1 or 5.

## Revendications

1. Procédé de préparation de mélanges de polyamines aromatiques contenant des composés de formule générale (I)
H₂N-A-CH₂-B-NH₂ (I)
dans laquelle A et B sont des résidus 1,4-phénylène, dont chacun, indépendamment de l'autre, peut avoir de 1 à 4 substituants choisis parmi les radicaux alkyle en C₁₋₂₀ et les atomes d'halogène,
par réaction d'un composé de formule générale (IV)
H-A-NH-CH₂-HN-B-H (IV)
et/ou d'un composé de formule générale (V)
H-A-NH-CH₂-B-NH₂ (V)
dans laquelle A et B sont substitués comme ci-dessus,
à une température comprise entre 20 et 200°C, en présence d'un catalyseur inorganique hétérogène qui est choisi parmi un ou plusieurs oxydes des éléments du 3ème au 10ème et de préférence du 4ème au 6ème Groupes du Tableau Périodique des Eléments, à l'exception du vanadium, et qui peut être activé par un acide.

2. Procédé selon la revendication 1, **caractérisé en ce que** le composé ayant la formule générale (IV) ou (V) est obtenue par réaction, avec du formaldéhyde ou des précurseurs du formaldéhyde, d'une aniline éventuellement substituée sur le noyau aromatique sur la position o ou m par 1 à 4 substituants choisis parmi les radicaux alkyle en C₁₋₂₀ et les atomes d'halogène.

3. Procédé de préparation de mélanges de polyamines aromatiques contenant des composés de formule générale (I)
H₂N-A-CH₂-B-NH₂ (I)
dans laquelle A et B sont des résidus 1,4-phénylène, dont chacun, indépendamment de l'autre, peut avoir de 1 à 4 substituants choisis parmi les radicaux alkyle en C₁₋₂₀ et les atomes d'halogène,
par réaction, avec du formaldéhyde ou des précurseurs du formaldéhyde, d'anilines éventuellement substituées sur le noyau aromatique sur la position o ou m par 1 à 4 substituants choisis parmi les radicaux alkyle en C₁₋₂₀ et les atomes d'halogène, à une température comprise entre 20 et 200°C, en présence d'un catalyseur inorganique hétérogène qui est choisi parmi un ou plusieurs oxydes des éléments du 3ème au 10ème et de préférence du 4ème au 6ème Groupes du Tableau Périodique des Eléments, à l'exception du vanadium, et qui peut être activé par un acide.

4. Procédé selon la revendication 2 ou 3, **caractérisé en ce que** l'eau qui se forme lors de la réaction est éliminée en continu.

5. Procédé selon l'une des revendications 1 à 4, **caractérisé en ce que** les noyaux aromatiques ne sont pas substitués sur au moins une position o par rapport au groupe amino, et les mélanges de polyamines aromatiques contiennent en outre des composés de formule générale (II) et/ou (III)
H₂N-D-CH₂-B-NH₂ (II)
dans laquelle D est un résidu 1,2-phénylène et B est un résidu 1,4-phénylène, qui peuvent être substitués par des substituants selon la revendication 3,
H₂N-D-CH₂-E-NH₂ (III)
dans laquelle D et E sont des résidus 1,2-phénylène pouvant être substitués par des substituants selon la revendication 3.

6. Procédé selon la revendication 2, 3 ou 4, **caractérisé en ce qu'**on utilise de l'aniline ou de l'o-toluidine.

7. Procédé selon l'une des revendications 1 à 6, **caractérisé en ce qu'**on utilise en tant que catalyseur un ou plusieurs oxydes d'éléments du 4ème au 6ème Groupes du Tableau Périodique des Eléments, le catalyseur pouvant être activé par un acide.

8. Procédé selon l'une des revendications 1 à 7, **caractérisé en ce qu'**on utilise en tant que catalyseur du dioxyde de titane, de l'oxyde de tungstène, de l'oxyde de molybdène, du dioxyde de zirconium ou des mélanges de ces oxydes, le catalyseur pouvant être activé par un acide.

9. Procédé selon l'une des revendications 1 à 8, **caractérisé en ce que** le composé de formule (I) se forme en tant que produit principal.

10. Procédé selon l'une des revendications 1 à 9, **caractérisé en ce que** la réaction est mise en oeuvre en l'absence de solvant.

11. Utilisation d'un catalyseur inorganique hétérogène choisi parmi un ou plusieurs oxydes des éléments du 3ème au 10ème Groupes du Tableau Périodique des Eléments, à l'exception du vanadium, et qui peut être activé par un acide ;
lors de la préparation de mélanges de polyamines aromatiques tels que définis dans la revendication 1 ou 5.
